(19) Europäisches Patentamt
European Patent Office
Office européen des brevets

(11) Publication number: **0 427 484 B1**

(12) **EUROPEAN PATENT SPECIFICATION**

(45) Date of publication of patent specification: **20.07.94**

(51) Int. Cl.5: **C07D 263/58**, A01N 43/76, A61K 31/42

(21) Application number: **90312067.3**

(22) Date of filing: **02.11.90**

The file contains technical information submitted after the application was filed and not included in this specification

(54) **Novel benzoxazolone compounds and the use thereof as microbicides.**

(30) Priority: **09.11.89 US 433862**

(43) Date of publication of application:
**15.05.91 Bulletin 91/20**

(45) Publication of the grant of the patent:
**20.07.94 Bulletin 94/29**

(84) Designated Contracting States:
**AT BE CH DE DK ES FR GB GR IT LI LU NL SE**

(56) References cited:
**EP-A- 0 170 191**
**EP-A- 0 311 135**

**CHEMICAL ABSTRACTS, vol. 97, no. 3, July 19, 1982, Columbus, Ohio, USA SKACANI,IVAN et al. "6-Substituted 3-(3-iodo- propargyl)-2-(3H)-benzo- thiazolones"page 713, column 1, abstract- -no. 32 779c**

**CHEMCIAL ABSTRACTS, vol. 77, no. 17, October 23, 1972, Columbus, Ohio, USA**

(73) Proprietor: **ROHM AND HAAS COMPANY**
**Independence Mall West**
**Philadelphia Pennsylvania 19105(US)**

(72) Inventor: **Chi-Tung Hsu, Adam**
**1686 Heebner Eay**
**Lansdale, Pennsylvania 19446(US)**

(74) Representative: **Angell, David Whilton et al**
**ROHM AND HAAS**
**European Operations Patent Department**
**Lennig House**
**2 Mason's Avenue**
**Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**LINDQUIST, AKE et al. "Acetylene compounds of potential pharmacological value.XVII. N-(4-Tertiary amino-2-butynyl)-substituted bicyclic lactams" page 433,column 1, abstract- -no. 114 211q**

**CHEMICAL ABSTRACTS, vol. 91, no. 19, November 5, 1979, Columbus, Qhio, USAAB-DULLAEV, KH. et al. "Results of a search for antitrichomonas drugs amongorganic acid, urea, benzoxazolinone, pyrazole, heterocyclic thione, andaromatic sulfonic acid ester derivatives" page 25, column 1, abstract- -no. 151104s**

## Description

This invention concerns novel benzoxazolones containing a halosubstituted alkyne (halopropargyl) group, and the use of such compounds as microbicides. More specifically, the compounds to which the invention is directed have the formula (I)

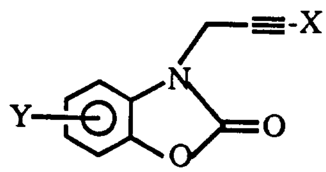

(I)

where

X = I or Br, and

Y = H, halogen, $NO_2$, $(C_1-C_4)$ alkyl, $(C_1-C_4)$ alkoxy, CN, OCO $(C_1-C_4)$alkyl, OCOPh or halo $(C_1-C_4)$ alkyl and may comprise one or two substituents.

As used hereinafter the term "microbicide" (or "biocide") is intended to include, but is not restricted to, bactericides, fungicides and algicides, and microbicidal activity refers to both the elimination of and also to the inhibition or prevention of growth of microbial organisms such as bacteria, fungi and algae.

Compounds having the formula

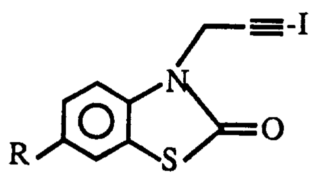

where R is H, Cl or $NO_2$ are disclosed in CZ-A-194354 (Chem Abs. 97:23779c), and are said to have antifungal activity. EP-A-230874 discloses related compounds having the formula

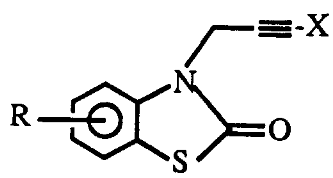

where X = H or Br: these compounds are said to have herbicidal activity. Neither document mentions benzoxazolones.

Benzoxazolones having the formula

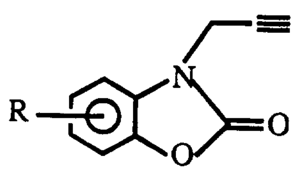

are disclosed in J. Pharm. Sc., 57, 1763 (1968), Chem. Abs. 89: 179902h and Chem abs 94:192196d; however no microbicidal activity is mentioned in any of the disclosures. Chem Abs 94: 192196d also discloses a benzoxazolone of the formula

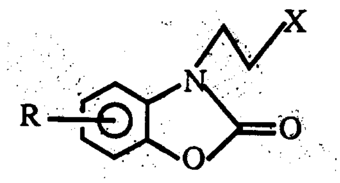

where X = CN, $CONH_2$, COHNPh, and states that this compound has antifungal activity. It should be noted that this compound does not possess a propargyl group.

In a first aspect the present invention provides compound of the formula (I)

(I)

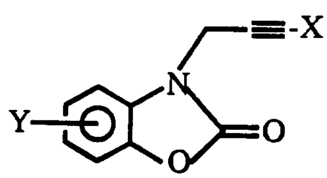

wherein

X is I or Br; and

Y comprises one or two substituents each of which is independently H, halogen, ($C_1$-$C_4$) alkyl, ($C_1$-$C_4$) alkoxy, CN, OCO($C_1$-$C_4$)alkyl, OCOPh, or halo ($C_1$-$C_4$) alkyl.

Another aspect of the invention relates to the use of the compounds, or a composition containing said compounds, as a microbicide, and to microbicidal compositions containing the compounds.

A further aspect of the invention provides a process for preparing such compounds, which comprises reacting a compound of the formula (III)

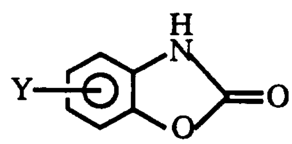

(III)

with a propargyl halide $X^1$-$CH_1$-C≡C-X where $X^1$ is Br or Cl and X is I, Br or H, and then in the case where X is H halogenating the resulting product to produce a compound of the invention. When X is Br or I, the propargyl halide is made by halogenating a compound of the formula $X^1$-$CH_2$-C≡CH and then reacting the halogenated product directly with compound (III) above.

As stated above, the compounds of the invention have the formula (I)

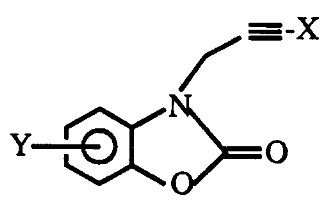

(I)

where

X = I or Br, and

Y = halogen, H, $NO_2$, ($C_1$-$C_4$) alkyl, ($C_1$-$C_4$)alkoxy, CN, OCO ($C_1$-$C_4$)alkyl, OCOPh or halo ($C_1$-$C_4$) alkyl.

Y may comprise a single substituent on the aromatic ring; alternatively the ring may be disubstituted, with the Y substituents being the same or different.

It is preferable that X is iodine: compounds in which Y is selected from H, chlorine and $NO_2$ are also preferred. Particularly preferred compounds include 3-(3-iodopropargyl)-2-benzoxazolone.

Compositions comprising a compound according to formula I and either an agronomically acceptable carrier, a cosmetic agent, a cutting oil, a soap or synthetic detergent, a stabilizer, a film forming material, or the like have a wide range of utility for protecting against or controlling microorganisms from a wide variety

of classes including fungus, bacteria, algae, viruses and yeasts. The preferred utilities of the compositions are to protect wood, paint, adhesive, glue, paper, textile, leather, plastics, cardboard, lubricants, cosmetics, food, caulking, feed and industrial cooling water from microorganisms.

The invention also provides a method of inhibiting or preventing the growth of bacteria, fungi or algae in a locus subject or susceptible to contamination thereby, comprising incorporating into or onto the locus a composition containing a compound according to any of claims 1 to 4 in an amount effective to adversely affect the growth of said bacteria, fungi or algae.

The following lists specific industries and applications of the compounds or compositions:

| Industry | Application |
|---|---|
| Adhesives, Sealants | adhesives<br>caulks<br>sealants |
| Agriculture/food chain | adjuvant preservation<br>agricultural active ingredient<br>agricultural chemical preservative<br>agricultural formulations preservation<br>animal feed preservation<br>dairy chemicals<br>fertilizer preservation<br>food preservation<br>food processing chemicals |

| | |
|---|---|
| | grain preservation<br>post-harvest produce protection<br>sugar processing<br>tobacco |
| Construction products | asphalt / concrete<br>cement modifiers<br>construction products<br>roof mastics<br>synthetic stucco<br>wall mastics<br>joint cement |
| Cosmetics and toiletries | cosmetics<br>raw materials for cosmetics, toiletries<br>toiletries |
| Disinfectants, antiseptics | antiseptic<br>disinfectant |
| Emulsions, dispersions | aqueous dispersions<br>dispersed pigments<br>latex<br>photographic emulsions<br>pigment slurries<br>polymer latices |
| Formulated household products | fabric softeners<br>polishes<br>waxes<br>hand dish detergents<br>raw materials<br>liquid detergents<br>hand soaps |
| Industrial processing, misc | electrodeposition paint, baths, rinses.<br>electrodeposition pre-treatment, post rinses<br>industrial fluids preservation<br>pasteurization baths<br>process aid preservation |
| Industrial water treatment | air washers<br>cooling towers |

| | |
|---|---|
| | cooling water<br>water cooling<br>preservation/treatment of wooden cooling tower slats and structural members<br>can warmers<br>brewery pasteurization<br>closed loop water cooling systems |
| Laundry | household laundry products<br>laundered goods<br>laundry wash water<br>sanitizers-laundry |
| Leather, Leather products | leather and hide<br>leather and hide products |
| Lubricants, hydraulic aids | automotive lubricants and fluids<br>conveyor lubricants<br>greases<br>hydraulic fluids<br>lubricants |
| Medical devices | diagnostic enzymes<br>diagnostic kits<br>medical devices |
| Metalworking & related app's | cutting fluids<br>metal cleaning<br>metalworking fluids |
| Odor control (active ingredient) | air conditioning<br>animal bedding<br>cat litter<br>chemical toilet prep'ns<br>deodorizers<br>humidifiers<br>industrial deodorants<br>sanitary formulations<br>toilet bowls |
| Paints and coatings coating | emulsions<br>paints |

| | |
|---|---|
| Paper and wood pulp, their products | absorbant materials of paper and wood pulp<br>packaging materials of paper and wood pulp<br>paper<br>paper products<br>paper treatment<br>soap wrap<br>wood pulp<br>wood pulp products |
| Paper mill | paper mill slimicides<br>pulp and paper slurries |
| Petroleum refining, fuels | aviation fuels (jet fuel, aviation gas)<br>crude oils<br>burner, diesel and turbine fuel oils<br>coal slurries<br>diesel fuel additives<br>diesel fuels<br>fuels<br>gasoline<br>heating oils<br>hydrocarbons<br>kerosene<br>liquefied petroleum gas<br>petrochemical feedstocks<br>petroleum products, storage, transportation and production<br>recycled petroleum products<br>residual fuel oils<br>turbine oils |
| Photographic Chemicals and process | photographic processing - wash water, rinses<br>photoprocessing<br>photoplate processing chemicals (developers, stabilizers etc) |
| Printing | fountain solutions (printing)<br>ink components (pigments, resins, solvents, etc)<br>inks |

| | |
|---|---|
| sanitizers (active) | sanitizers<br>sanitizers-dairy<br>sanitizers-dental<br>sanitizers-fermentation<br>sanitizers-food preparation<br>sanitizers-food processing<br>sanitizers-medical<br>sanitizers-rendering<br>sanitizers-veterinary |
| Soaps, detergents, cleaners | cleaners<br>detergents<br>household cleaners<br>industrial cleaners<br>liquid soaps<br>oil and grease remover<br>powdered soaps<br>raw materials for cleaning products<br>soaps<br>surfactants |
| Textiles, textile products | bonded fabrics<br>burlap<br>canvas<br>canvas goods<br>carpet backing<br>carpets<br>clothing<br>coated fabrics<br>curtains<br>draperies<br>engineering textiles<br>fibers<br>geotextiles<br>goods made of textiles<br>knitted fabrics<br>nets<br>nonwoven fabrics<br>rope<br>rugs<br>textile accessories<br>textile products<br>textiles |

| | |
|---|---|
| | upholstery |
| | woven fabrics |
| | yarn |
| Textile processing | dye fixatives |
| | dyes |
| | fiber lubricants |
| | hand modifiers |
| | sizes |
| | textile processing fluids |
| Therapeutic (active or preservative) | animal health/veterinary |
| | aquaculture |
| | dental |
| | human health |
| | pharmaceutical /therapeutic |
| Water purification | charcoal beds |
| | deionization resins |
| | filters |
| | membranes |
| | reverse osmosis membranes |
| | ultrafilters |
| | water purification |
| | water purification pipes, tubing |
| Wood applications | lazures (wood stains) |
| | wood |
| | wood products |
| Miscellaneous | alcohols |
| | bedding incorporating water or gels |
| | ceramic |
| | contact lens cases-leaching |
| | electronic circuitry |
| | electronics chemicals |
| | enzymes-food production |
| | enzymes |
| | enzymes-industrial |
| | gel cushions |
| | marine antifoulants |
| | mildewcides |
| | wood |
| | plastics |

laundry
mining
natural rubber latex
oil field injection waters including enhanced recover injection fluids, drilling, fracturing and completion fluids
pipes
plastics
polymer systems
polymers and resins (synthetic and natural)
reagent preservation
rubber
rubber products
skin remover
solid protective/decorative films
stains
swimming pools
waste treatment
water beds

The amounts of the compound to be used depend on the application. The useful amounts for a particular application are similar to amounts used for other microbicide compounds. Typically, a composition may contain anything from 0.001 to 99.99% by weight of the compound; more usually from 0.01 to 5%.

The compound can be used in combination with other microbicides. The term "microbicide" is considered equivalent to "antimicrobial" as used herein.

The compounds (I) of the present invention may be prepared by the following reaction:

(II)

(I)

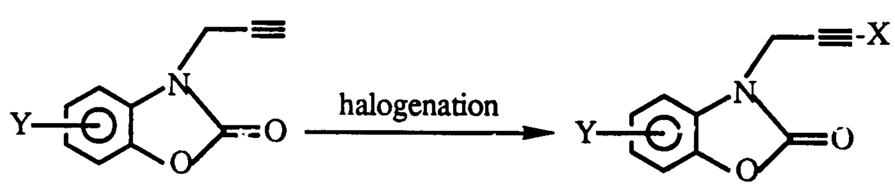


A variety of halogenation reactions may be utilized to prepare compounds of formula (I). One useful method is described here. A solution or suspension of compounds of formula (II) in solvents such as acetone, methyl ethyl ketone, or tetrahydrofuran is allowed to react with N-bromosuccinimide or N-iodosuccinimide in the presence of a catalyst such as silver nitrate. The reaction usually takes place within a range of from 20 min. to 24 hrs. The reaction is usually conducted at a temperature of between 0 °C and 25 °C.

The compounds of formula (II) may be prepared by the following reaction:

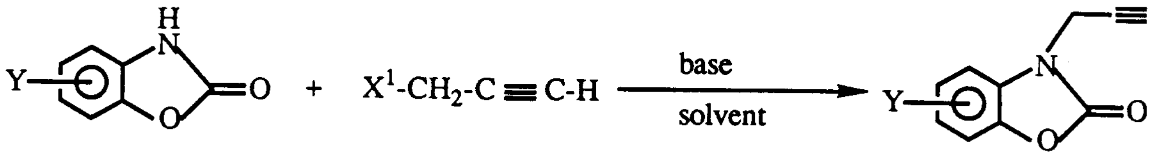


(III) (IV) (II)

$X^1$ = Br or Cl

A solution or suspension of a compound of formula (III) in a solvent such as acetone or methyl ethyl ketone is treated with a base such as potassium carbonate or sodium carbonate followed by a propargylating agent such as propargyl bromide or propargyl chloride of formula (IV). The above treatment is usually conducted at room temperature under inert atmosphere such as nitrogen with stirring. The reaction mixture is then refluxed for a time ranging from 1 hr to 24 hr depending on the particular identity of compound (III). The reaction mixture is then cooled to room temperature and worked-up in a standard way. The reagents of formula (IV) are generally commercially available or can be prepared by using methods described in literature. The starting materials of formula (III) are also commercially available or can be prepared by using methods described in literature.

The compounds of the invention can also be prepared in one step from compounds of formula (III) by reacting them with a halopropargylating agent such as 3-iodopropargyl bromide or 3-iodopropargyl chloride in the presence of a base such as sodium carbonate or potassium carbonate. The reaction sequence may be depicted as follows:

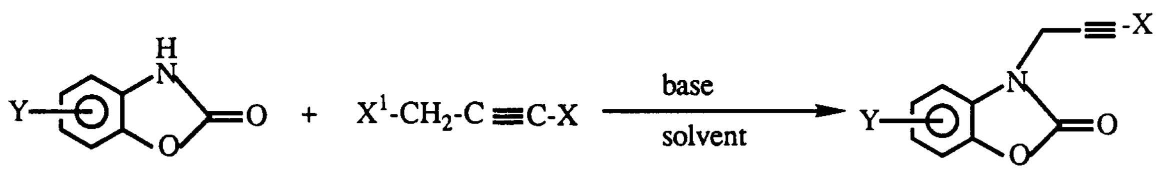


(III) (V) (I)

The compounds of formula (V) can be prepared from a method described in literature as follows:

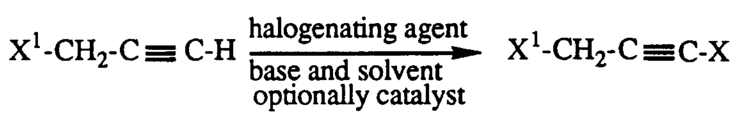


(IV) (V)

The following example describes the preparation of a compound of the invention, 3-(3-iodopropargyl)-2-benzoxazolone.

Example 1

In a 2 liter round bottom flask, equipped with a mechanical stirrer, a condenser and a drying tube on the top of the condenser, was placed benzoxazolone (20 g, 0.148 mole), dry acetone (575 ml), anhydrous potassium carbonate (40.9 g, 0.296 mole), and propargyl bromide (80% in toluene, 26 g, 0.175 mole). The reaction mixture was refluxed for 24 hr with stirring. The reaction mixture was cooled down to room temperature and filtered, and the filtrate concentrated to a brown residue. After crystallization with methylene chloride/hexane, a tan solid was obtained yielding 22.8 g (89%). m.p. 96-98 °C. An NMR spectrum showed the desired intermediate, 3-propargyl-benzoxazol-2-one.

To the solution of 3-propargyl-benzoxazol-2-one (7 g, 0.04 mole) in dry acetone (100 ml) in a 300-ml round bottom flask with magnetic stirring was added N-iodosuccinimide (11.1 g, 0.048 mole), followed by

silver nitrate (0.3 g, 0.00176 mole). The reaction mixture was stirred at room temperature for 1 hr. The solid was filtered off and the filtrate concentrated on a rotary evaporator to give a residue. The crude solid was dissolved in ethyl acetate and washed with water and brine. The organic layer was dried over magnesium sulfate. After filtering the drying agent and evaporating the solvent, the resultant solid was crystallized from hexane/ethyl acetate yielding 9.1 g (75.8%) white solid. m.p. 162-164 °C. An NMR spectrum showed the desired compound.

The following examples demonstrate the microbicidal activity of certain compounds of the invention: 3-(3-iodopropargyl)-2-benzoxazolone ($Y = H_1 X = I$), 3-(3-iodopropargyl)-6-chloro-2-benzoxazolone (Y = Cl,X = I), 3-(3-bromopropargyl)-6-chloro-2-benzoxazolone (Y = Cl,X = Br) and 3-(3-bromopropargyl)-2-benzoxazolone (Y = H, X = Br).

Example 2

In vitro bactericidal and fungicidal evaluations

Activity against certain bacteria and fungi was determined using a minimum inhibitory concentration (MIC) test, which establishes the minimum concentration of test compound required to achieve complete inhibition of growth of the test organism.

An MIC value was obtained using a broth, two-fold serial dilution test performed as follows: A stock solution or dispersion of the test compound at a concentration of 1%, was made in a 5:3:2 solvent solution of acetone, methanol, and water. A volume of the stock solution was dispensed into culture media to give an initial starting test concentration of 500 ppm compound.

When the test was ready to be done, each vessel in the dilution series, except the first vessel, contained an equal volume of compound free broth. The first vessel contained twice the volume of broth with the starting concentration of test compound. One half of the broth from the first vessel was transferred to the second vessel. After being mixed, one half the resulting volume was removed from the second vessel and transferred to the third vessel. The entire cycle was repeated sufficiently to give a series of concentrations amounting to 500, 250, 125, 63, 31, 16, 8, and 4 ppm (or 25, 12.5, 6.2, 3.1, 1.6, 0.8, 0.4, 0.2), respectively.

Each vessel was then inoculated with a cell suspension of the appropriate test organism. Bacteria were grown in broth and fungi on agar slants for a time and at a temperature appropriate to the species being tested. At the end of the growth period, the bacteria containing broth was vortexed to disperse the cells. In the case of fungi, the spores were harvested by pipetting water onto the slant and dislodging the spores with a sterile loop. The cell/spore suspension was standardized by controlling incubation time, temperature, and the volume of the diluent. The suspension was then used to inoculate the vessels containing the broth compound. The vessels were then incubated at the appropriate temperature. Alter the incubation, the vessels were examined for growth/no growth. The minimum inhibitory concentration (MIC) is defined as the lowest concentration of compound that results in complete inhibition of growth of the test organism.

The organisms tested were bacteria Pseudomonas fluorescens (Ps.fl) - ATCC 948, Pseudomonas aeruginosa (Ps.ae) - ATCC 15442, Escherichia Coli (E.coli) - ATCC 11229, and Staphylococcus aureus (S.aur) - ATCC 6538; and fungi Aspergillus niger (A. niger) - ATCC 6275, Penicillium funiculosum (P. fun.) - ATCC 11797, Cladosporium resinae (A. res.) - ATCC 52833, Aureobasidium pullulans (A. pull.) - ATCC 9248, Cloeophyllum trabeuor (G. trab.) - ATCC 11539; and two yeasts Sacharomyces cereresae (S. cer.) - ATCC 560, and Rhorotorularubra (R. rubra) - ATCC 2503.

| Results | MIC (ppm) for each compound | | | |
|---|---|---|---|---|
| ORGANISM | Y = H,X = I | Y = 6-Cl, X = I | Y = 6-Cl, X = Br | Y = H, X = Br |
| Ps.fl | >250 | 16 | >250 | >250 |
| Ps.ae | >250 | >250 | >250 | >250 |
| E.coli | >250 | >250 | >250 | >250 |
| S. aur | >250 | 16 | >250 | >250 |
| A. niger | <0.13 | <0.13 | 32 | > 25 |
| A. pull | 0.25 | ≦ 1 | 32 | > 25 |
| P. fun | ≦ 1 | ≦ 1 | > 25 | > 25 |
| Cl.res. | ≦ 5 | ≦ 1 | > 25 | > 25 |
| G. trab. | ≦0.2 | ≦ 1 | > 25 | > 25 |
| S.cer | ≦ 1 | ≦ 5 | > 25 | > 25 |
| R. rubra | ≦ 1 | ≦ 1 | > 25 | > 25 |

Example 3

In vitro plant fungicidal tests

The effectiveness of three of the four compounds tested above at controlling selected plant fungi was tested by the following method.

The organisms employed in the test were:

PYU Pythium ultimum (Oomycete)
PHY Phytophthora capsici (Oomycete)
PIR Piricularia oryzae (Ascomycete)
HEL Cochliobolus sativus (Ascomycete)
BOC Botrytis cinerea (Ascomycete)
FUS Fusarium roseum (Ascomycete)
SEP Septoria nodorum (Ascomycete)
RHI Rhizoctonia solani (Basidiomycete)
XAN Xanthomonas campestris (bacterium)

Method:

1. Culture maintenance: All 8 fungi and the bacterium used in this test were transferred and maintained on potato dextrose agar plates (2 plates/organism). Organisms were used at the following ages: a. 1 week old: PYU, PHY, RHI; b. 2 weeks old: XAN, PIR, BOC, HEL, FUS, SEP, COL, MON, CER, UST, ALT; c. 3 weeks old: PSH, VEN. Pythium ultimum and Phytophthora capsici were transferred to asparagine-sucrose broth shake cultures (ASB). Rhizoctonia solani, Fusarium roseum, and Zanthomonas campestris were maintained in yeast extract-dextrose broth (YDB) on a shaker. Culture flasks were inoculated with 6 mycelial plugs each (except for Pythium which was inoculated with only 3 plugs) taken from PDA plates. All liquid shaker cultures were used after 2 days growth.

2. Inoculum preparation. Conidia and mycelium from PIR, BOC, HEL, SEP, COL, MON, CER, PSH, UST and ALT were lightly scraped off into YDB so that mostly conidia are used as inoculum. XAN broth culture was poured (1 ml culture/100 ml broth) into YDB. PYU, PHY, RHI and FUS cultures were ground up and all but Pythium and Phytophthora filtered. Ten ml of the culture solutions of R. solani and F. roseum were added to 90 ml of YDB and 10 ml of the P. capsici was added to 90 ml ASB. Two ml of the culture solution of P. ultimum was added to 98 ml of ASB. 175 µl (single dose) or 100µl (dose-response test) of inoculum broth was placed in each well of microtiter plates. The plates with inoculated media were refrigerated overnight.

3. Addition of test compound. 10 mg a.i. of the test compound was placed in 1 ml 1:1 acetone:methanol. 5 microliters of this solution was pipetted into the microtiter plates containing 245 microliters of sterile water according to the grid. 25 microliters of the resulting solution was transferred to the inoculated plates. The results below are reported as % disease control relative to untreated check plants.

| Compound | Dose (ppm) | % DISEASE CONTROL | | | | | | | | |
|---|---|---|---|---|---|---|---|---|---|---|
| | | BOC | FUS | HEL | PHY | PIR | PRU | RMI | SEP | XAN |
| Y = H, X = I | 25 | 100 | 100 | 100 | 100 | 100 | 100 | 95 | 100 | 0 |
| Y = 6-Cl, X = I | 25 | 100 | 100 | 100 | 100 | 100 | 95 | 100 | 100 | 0 |
| Y = 6-Cl, X = Br | 25 | 50 | 0 | 50 | 0 | 0 | 50 | 0 | 0 | 0 |

Example 4

In vivo plant disease control tests

3-(3-iodopropargyl)-2-benzoxazolone was tested for fungicidal activity in vivo against cucumber downy mildew (CDM), rice blast (RB), wheat glume blotch (WGB), tomato late blight (TLB), wheat powdery mildew (WPM), wheat stem rust (WSR) and wheat leaf rust (WLR). In tests on cereals (except for rice plants used for testing rice blast), the plants were trimmed about 24 hours prior to the application of the fungicide compound to provide a uniform plant height and to facilitate uniform application of the compound and inoculation with the fungus. The test compound was dissolved in a 2:1:1 mixture of water, acetone, and methanol, sprayed onto the plants, allowed to dry (four to six hours), and then the plants were inoculated with the fungus. Each test utilized control plants which were sprayed with the water, acetone, and methanol mixture and inoculated with the fungus. The remainder of the technique of each of the tests is given below and the results are reported as percent disease control (percentages of plants treated with the compounds of the present invention lacking disease signs or symptoms compared to the untreated control plants).

Cucumber Downy Mildew (CDM):

Pseudoperonospora cubensis was maintained on leaves of live Marketer cucumber plants in a constant temperature room at 65°F to 75°F in humid air with moderate light intensity for 7 to 8 days. A water suspension of the spores from infested leaves was obtained and the spore concentration was adjusted to about 100,000 per ml of water.

Marketer cucumber seedlings were inoculated by spraying the underside of the leaves with a DeVilbiss atomizer until small droplets were observed on the leaves. The inoculated plants were incubated in a mist chamber for 24 hours at about 70°F and then subsequently incubated for 6 to 7 days in a controlled temperature room under mist at 65°F to 75°F. Seven days after inoculation, the percent disease control was determined.

Rice Blast (RB):

Nato rice plants were inoculated with Piricularia oryzae (about 20,000 conidia per ml) by spraying the leaves and stems with an airbrush until a uniform film of inoculum was observed on the leaves. The inoculated plants were incubated in a humid environment (75°F to 85°F) for about 24 hours, then placed in a greenhouse environment (70°F to 75°F). Seven to eight days after inoculation, the percent disease control was determined.

Wheat Glume Blotch (WGB):

Septoria nodorum was produced by incubating plates containing sporulating mycelium for 48 hours in darkness at 20°C until a white mycelium was observed. The plates were then incubated at 21°C under continuous light for 15-20 days, at which stage the mycelium had a pink color.

A suspension of spores in deionized water was adjusted to a concentration of 150-300 spores/ml. Wheat plates were inoculated by spraying the leaves with the spore suspension using a hand sprayer, optionally after spraying the plants with a light mineral oil and waiting 5 minutes. The inoculated plants were incubated for 72 hours in a humidity cabinet at 20°C with a photoperiod of 16 hours light/8 hours dark. The plants were then placed in a growth chamber for 7-9 days at 20°C with 16 hours light/8 hours dark, before percent disease control was evaluated.

Tomato Late Blight (TLB):

Phytophthora infestans was cultured on four week old Pixie tomato plants in a controlled environment room (65°F to 70°F and 100% relative humidity). After storage, the spores were washed from the leaves with water and dispersed by DeVilbiss atomizer over three week old Pixie tomato plants which had been sprayed previously with experimental fungicides. The inoculated plants were placed in a humidity cabinet at 70°F and constant mist for 24 hours for infection. The plants were then moved to the controlled environment room as above and scored after three more days incubation. Disease control levels were recorded as percent control four days after inoculation and five days after spraying the compounds.

Wheat Powdery Mildew (WPM):

Erysiphe graminis (f. sp. tritici) was cultured on Pennol wheat seedlings in a controlled temperature room at 65°F to 75°F. Mildew spores were shaken from the culture plants onto Pennol wheat seedlings which had been sprayed previously with the fungicide compound. The inoculated seedlings were kept in a controlled temperature room at 65°F to 75°F and subirrigated. The percent disease control was rated 8 to 10 days after the inoculation.

Wheat Stem Rust (WSR):

Puccinia graminis (f. sp. tritici Race 15B-2) was cultured on Wanzer wheat seedlings for a period of 14 days in a greenhouse. A water suspension of the spores from infested plants was obtained and the spore concentration was adjusted to about 200,000 spores per ml of deionized water. Wanzer wheat plants which had been previously treated with the fungicide compounds were inoculated by applying the stem rust spore suspension, until runoff, with a DeVilbiss atomizer at 5 lbs. per square inch air pressure. Alter inoculation, the plants were placed in a humid environment at approximately 75°F where they were exposed to 12 hours of continuous darkness followed by a minimum of 3 to 4 hours of light having an intensity of about 500 footcandles. The temperature in the chamber did not exceed 85°F. At the end of the light period, the plants were placed in a greenhouse where they were permitted to grow for a period of two weeks at which time the percent disease control was determined.

Wheat Leaf Rust (WLR):

Puccinia recondita (f. sp. tritici Races PKB and PLD) was cultured on seven day old wheat (cultivar Fielder) over a 14 day period in the greenhouse. Spores were collected from the leaves with a cyclone vacuum or by settling on aluminum foil. The spores were cleaned by sieving through a 250 micron opening screen and stored or used fresh. Storage employed sealed bags in an Ultralow freezer. When stored, spores were heat shocked for two minutes at 40°F before use. A spore suspension was prepared from dry uredia by adding 20 mg (9.5 million) per ml of Soltrol oil. The suspension was dispensed into gelatin capsules (0.7 ml capacity) which attached to the oil atomizers. One capsule was used per flat of twenty of the two inch square pots of seven day old Fielder wheat. Alter waiting for at least 15 minutes for the oil to evaporate from the wheat leaves, the plants were placed in a dark mist chamber (18-20°C and 100% relative humidity) for 24 hours. The plants were then put in the greenhouse for the latent period and scored after 10 days for disease levels. Protective and curative tests were inoculated one day after and two days, respectively, before spraying the plants with the test chemicals.

| COMPOUND | DOSE(PPM) | % DISEASE CONTROL | | | | | |
|---|---|---|---|---|---|---|---|
| | | CDM | RB | WGB | TLB | WLR | WPM |
| Y=H, X=I | 200 | 50 | 95 | 0 | 0 | 90 | 90 |

## Claims

**1.** Compound of the formula (I)

(I) 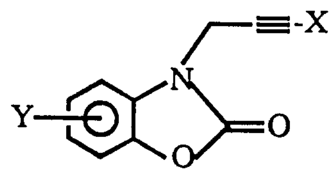

wherein
X is I or Br; and
Y comprises one or two substituents each of which is independently H, halogen, ($C_1$-$C_4$) alkyl, ($C_1$-$C_4$) alkoxy, CN, OCO($C_1$-$C_4$)alkyl, OCOPh, or halo ($C_1$-$C_4$) alkyl.

**2.** Compound according to claim 1 wherein X is I.

**3.** Compound according to claim 1 wherein Y is H or chlorine.

**4.** Compound according to claim 1 having the formula

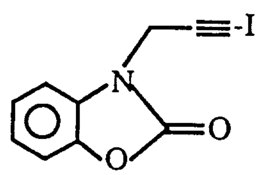

**5.** Composition comprising a compound according to claim 1 and a second component which is an agronomically acceptable carrier, a cosmetic agent, a cutting oil, a metal-working fluid, a soap or a synthetic detergent, a stabilizer, or a film-forming material.

**6.** Composition according to claim 5 wherein the compound is present in an amount of from 0.001 to 99.99% by weight, preferably from 0.01 to 5% by weight.

**7.** Process for preparing a compound as defined in any of claims 1 to 4 comprising reacting a compound of the formula (III)

(III) 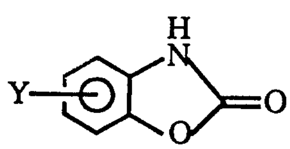

with a compound of the formula $X^1$-$CH_2$-C≡C-X, wherein $X^1$ is Br or Cl and X is I, Br or H, and then in the case where X is H further reacting the product thereof with an iodinating or brominating agent.

**8.** Process according to claim 7, wherein the compound of formula $X^1$-$CH_2$-C≡C-X, in the cases where X is Br or I, is made by reacting a compound of the formula $X^1$-$CH_2$-C≡CH with an iodinating or brominating agent.

**9.** The non-therapeutic use of a compound according to any of claims 1 to 4, or a composition according to claims 5 or 6, as a microbicide.

**10.** Non-therapeutic method of inhibiting or preventing the growth of bacteria, fungi or algae in a locus subject or susceptible to contamination thereby, comprising incorporating into or onto the locus a

composition containing a compound according to any of claims 1 to 4 in an amount effective to adversely affect the growth of said bacteria, fungi or algae.

**Patentansprüche**

1. Verbindung der Formel (I)

(I) 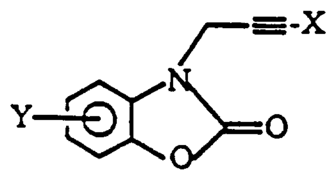

in der X Jod oder Brom ist und Y einen oder zwei Substituenten darstellt, von denen jeder unabhängig Wasserstoff, Halogen, $(C_1-C_4)$-Alkyl, $(C_1-C_4)$-Alkoxy, CN, $OCO(C_1-C_4)$-Alkyl, OCOPh oder $Halo(C_1-C_4)$-Alkyl ist.

2. Verbindung nach Anspruch 1, **dadurch gekennzeichnet**, daß X Jod ist.

3. Verbindung nach Anspruch 1, **dadurch gekennzeichnet**, daß Y Wasserstoff oder Chlor ist.

4. Verbindung nach Anspruch 1 mit der Formel

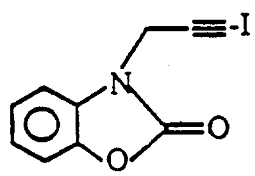

5. Zusammensetzung enthaltend eine Verbindung nach Anspruch 1 und einen zweiten Bestandteil, der ein landwirtschaftlich verträglicher Träger, ein kosmetisches Mittel, ein Schneidöl, eine Metallbearbeitungsflüssigkeit, eine Seife oder ein synthetisches Detergens, ein Stabilisator oder ein filmbildendes Material ist.

6. Zusammensetzung nach Anspruch 5, **dadurch gekennzeichnet**, daß die Verbindung in einer Menge von 0,001 bis 99,99 Gew.-%, vorzugsweise von 0,01 bis 5 Gew.-%, vorhanden ist.

7. Verfahren zum Herstellen einer Verbindung nach einem der Ansprüche 1-4 durch Umsetzen einer Verbindung der Formel (III)

(III) 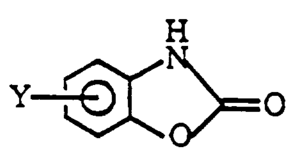

mit einer Verbindung der Formel $X^1$-$CH_2$-C≡C-X, in der $X^1$ Brom oder Chlor ist und X Jod, Brom oder Wasserstoff ist, und dann, wenn X Wasserstoff ist, weiteres Umsetzen des Reaktionsproduktes mit einem Jodierungsmittel oder Bromierungsmittel.

**8.** Verfahren nach Anspruch 7,
**dadurch gekennzeichnet**,
daß die Verbindung der Formel $X^1$-$CH_2$-C≡C-X in den Fällen, in denen X Brom oder Jod ist, hergestellt wird durch Umsetzen einer Verbindung der Formel $X^1$-$CH_2$-C≡CH mit einem Jodierungs- oder Bromierungsmittel.

**9.** Nicht-therapeutische Verwendung einer Verbindung nach einem der Ansprüche 1-4 oder einer Zusammensetzung nach Ansprüchen 5 oder 6 als Microbiozid.

**10.** Nicht-therapeutisches Verfahren zum Verhindern oder Hemmen des Wachstums von Bakterien, Pilzen oder Algen an einem Ort, der damit verunreinigt ist oder gegenüber Verunreinigung empfindlich ist durch Einbringen in den Ort oder auf die Stelle einer Zusammensetzung, die eine Verbindung nach einem der Ansprüche 1-4 enthalt, in einer wirksamen Menge, um das Wachstum dieser Bakterien, Pilze oder Algen gegenteilig zu beeinflussen.

## Revendications

**1.** Composé de formule (I)

(I) 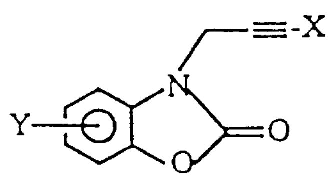

dans laquelle
X est I ou Br ; et
Y comprend un ou deux substituants, chacun d'eux étant, indépendamment, H, un atome d'halogène, un groupe alkyle en $C_1$-$C_4$, alcoxy en $C_1$-$C_4$, CN, OCO(alkyle en $C_1$-$C_4$), OCOPh ou halogéno-(alkyle en $C_1$-$C_4$).

**2.** Composé selon la revendication 1, dans lequel X est I.

**3.** Composé selon la revendication 1, dans lequel Y est H ou un atome de chlore.

**4.** Composé selon la revendication 1, de formule

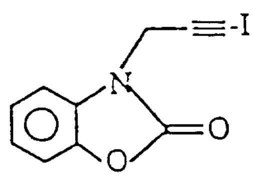

**5.** Composition comprenant un composé selon la revendication 1 et un second constituant, qui est un véhicule acceptable en agronomie, un agent cosmétique, une huile de coupe, un fluide métallurgique, un savon ou un détergent synthétique, un stabilisant ou une substance filmogène.

**6.** Composition selon la revendication 5, dans laquelle le composé est présent en une quantité de 0,001 à 99,99% en poids, de préférence de 0,01 à 5% en poids.

7. Procédé de préparation d'un composé tel que défini dans l'une quelconque des revendications 1 à 4, comprenant la réaction d'un composé de formule (III)

(III) 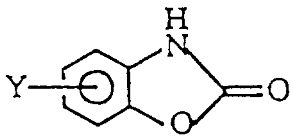

avec un composé de formule $X^1$-$CH_2$-C≡C-X, dans laquelle $X^1$ est Br ou Cl et X est I, Br ou H, puis, dans le cas où X est H, la réaction ultérieure du produit de cette réaction avec un agent d'iodation ou de bromation.

8. Procédé selon la revendication 7, dans lequel le composé de formule $X^1$-$CH_2$-C≡C-X, dans les cas où X est Br ou I, est préparé par réaction d'un composé de formule $X^1$-$CH_2$-C≡ CH avec un agent d'iodation ou de bromation.

9. Utilisation non thérapeutique d'un composé selon l'une quelconque des revendications 1 à 4, ou d'une composition selon les revendications 5 ou 6, comme microbicide.

10. Procédé non thérapeutique pour inhiber ou empêcher la croissance de bactéries, de champignons ou d'algues en un lieu sujet ou susceptible d'être contaminé par ces derniers, comprenant l'incorporation, dans ou sur le lieu, d'une composition contenant un composé selon l'une quelconque des revendications 1 à 4, en quantité efficace pour affecter négativement la croissance desdites bactéries, desdits champignons ou desdites algues.